# EUROPEAN PATENT APPLICATION

(11) **EP 3 943 592 A1**
(43) Date of publication of application: **26.01.2022**
(21) Application number: 19921625.0
(22) Date of filing: 18.10.2019
(51) Int. Cl.: C12M 1/34, C12Q 1/04, C12Q 1/66, G01N 1/00, G01N 1/10, G01N 21/76, G01N 21/11

(54) **MICROBIAL TEST KIT, MICROBIAL TEST METHOD AND MICROBIAL TEST DEVICE**

(30) Priority: 22.03.2019 JP 2019055355
(71) Applicant: Hitachi High-Tech Corporation, Minato-ku Tokyo 105-6409 (JP)
(72) Inventor: NODA Hideyuki, Tokyo 100-8280 (JP); ISHIMARU Masako, Tokyo 100-8280 (JP)
(74) Representative: MERH-IP Matias Erny Reichl Hoffmann Patentanwälte PartG mbB
(86) International application number: PCT/JP2019/041136
(87) International publication number: WO 2020/194813

(57) **Abstract**

Provided is a technique capable of easily performing a microbial test with high accuracy. A microbial test kit of the present disclosure includes: a first syringe capable of collecting a specimen and having a first syringe needle; a second syringe containing an ATP extraction reagent and having a second syringe needle; a sealed reaction container having a first opening, a first sealing member fitted to the first opening, a nozzle, a nozzle cap covering the nozzle, and a filter disposed so as to partition the first opening and the nozzle; a waste liquid container including a second opening and a second sealing member fitted to the second opening, and sealed under reduced pressure; and a luminescence measurement container that includes a third opening and a third sealing member fitted to the third opening, stores a luminescent reagent that emits light in presence of ATP, and is sealed under reduced pressure.

## Description

### Technical Field

The present disclosure relates to a microbial test kit, a microbial test method, and a microbial test device.

### Background Art

In a pharmaceutical factory or a beverage factory, products are manufactured in a manufacturing facility that realizes a sterile environment. Conventionally, a culturing method has been adopted in a microbial test for ensuring sterility of a production facility or a product. The culturing method is a method in which a specimen to which a culture medium is added is cultured in a thermostat for several days to 14 days, and the number of colonies of grown bacteria is visually counted. Therefore, it takes time to obtain the inspection result, and it is necessary to wait for the inspection result until the product is shipped. From such a background, it is desired to develop a microbial test method for quickly determining sterility.

One of the rapid and simple microbial test methods is Adenosine triphosphate (ATP) bioluminescence method (hereinafter, the method is referred to as an ATP method). The ATP method is a method for converting ATP present in a bacterial cell into light and measuring the light by using a luminescence reaction of a firefly. More specifically, luciferin and an ATP molecule are incorporated into luciferase, and the amount of luminescence when the oxidized luciferin (oxyluciferin) transitions from the excited state to the ground state with the consumption of ATP is measured. At this time, since the consumption of one molecule of ATP corresponds to generation of one photon, the number of generated photons is proportional to the number of ATPs. Since there are ATP molecules equivalent to 1 attomole (amol = 10⁻¹⁸ mol) as an energy source in one viable bacteria, the total number of viable bacteria can be estimated from the amount of luminescence by ATP in the specimen.

Since the luciferin-luciferase reaction has the most excellent quantum efficiency (Φ_{BL}: ≈ 0.5) in bioluminescence and chemiluminescence, one cell can be detected as photons corresponding to several 100,000 cells. Therefore, the ATP method is a method capable of detecting light corresponding to one cell in principle.

However, in order to detect an extremely small amount (several levels) of bacteria mixed in a specimen with high sensitivity and high accuracy, it is necessary not only to make the performance of the luminescence measurement device highly sensitive, but also to prevent contamination of ATP and bacteria present in the environment, microorganisms possessed by humans, dust adsorbing ATP, and the like. In general, ATP and free ATP derived from dead bacteria are mixed in the specimen, and thus, when the specimen is used as it is for luminescence measurement, it is not possible to accurately estimate the number of viable bacteria.

PTL 1 describes that in order to remove free ATP in a specimen, a specimen liquid is filtered, and microorganisms and dead bacteria are decomposed in advance using an ATP degrading enzyme such as apyrase.

### Citation List

### Patent Literature

PTL 1: WO 2016/147313

### Summary of Invention

### Technical Problem

However, in the ATP method including the method described in PTL 1, the reaction operation is generally complicated, and the inspection environment and the skill of the operator are likely to affect the inspection result. Therefore, due to contamination from the inspection environment or the operator, the inspection accuracy decreases, and false positives are likely to occur. Even if the aseptic operation is performed in the safety cabinet, it is difficult to completely prevent contamination, and particularly, in a negative/positive determination test such as the aseptic test, it is difficult to clearly prove whether the test is false positive or true positive.

Therefore, the present disclosure provides a technique capable of easily performing a highly accurate microbial test.

### Solution to Problem

A microbial test kit of the present disclosure includes: a first syringe capable of collecting a specimen and having a first syringe needle; a second syringe containing an ATP extraction reagent and having a second syringe needle; a sealed reaction container having a first opening, a first sealing member fitted to the first opening, a nozzle, a nozzle cap covering the nozzle, and a filter disposed so as to partition the first opening and the nozzle; a waste liquid container including a second opening and a second sealing member fitted to the second opening, and sealed under reduced pressure; and a luminescence measurement container that includes a third opening and a third sealing member fitted to the third opening, stores a luminescent reagent that emits light in presence of ATP, and is sealed under reduced pressure.

Other features of the present disclosure will be clear from the description and the accompanying drawings of this specification. In addition, embodiments of the present disclosure are achieved and realized by elements, combinations of various elements, the following detailed description, and the attached claims.

The description of this specification is given only as a typical example, and does not limit the scope of claims or applications of the present disclosure.

### Advantageous Effects of Invention

According to the microbial test kit of the present disclosure, highly accurate microbial test can be easily performed.

Objects, configurations, and effects besides the above description will be apparent through the explanation on the following embodiments.

### Brief Description of Drawings

FIG. 1 is a schematic diagram illustrating a reaction bottle of a microbial test kit according to a first embodiment.
FIG. 2 is a schematic diagram illustrating a waste liquid bottle of the microbial test kit according to the first embodiment.
FIG. 3 is a schematic diagram illustrating a luminescence measurement container of the microbial test kit according to the first embodiment.
FIG. 4A is a schematic diagram illustrating a sampling syringe of the microbial test kit according to the first embodiment.
FIG. 4B is a schematic diagram illustrating a reagent-filled syringe of the microbial test kit according to the first embodiment.
FIG. 5A is a diagram illustrating a microbial test method using the microbial test kit according to the first embodiment.
FIG. 5B is a diagram illustrating the microbial test method using the microbial test kit according to the first embodiment.
FIG. 5C is a diagram illustrating the microbial test method using the microbial test kit according to the first embodiment.
FIG. 5D is a diagram illustrating the microbial test method using the microbial test kit according to the first embodiment.
FIG. 5E is a diagram illustrating the microbial test method using the microbial test kit according to the first embodiment.
FIG. 5F is a diagram illustrating the microbial test method using the microbial test kit according to the first embodiment.
FIG. 5G is a diagram illustrating the microbial test method using the microbial test kit according to the first embodiment.
FIG. 5H is a diagram illustrating the microbial test method using the microbial test kit according to the first embodiment.
FIG. 5I is a diagram illustrating the microbial test method using the microbial test kit according to the first embodiment.
FIG. 6 is a flowchart illustrating an example of a microbial test method using the microbial test kit according to the first embodiment.
FIG. 7 is a diagram illustrating a photon counting time course.
FIG. 8 is a schematic diagram illustrating a configuration of a microbial test device according to a fourth embodiment.
FIG. 9 is a schematic diagram illustrating a configuration of a microbial test device according to a fifth embodiment.

### Description of Embodiments

Hereinafter, embodiments of the present disclosure will be described with reference to the accompanying drawings. However, it should be noted that each embodiment is merely an example for realizing the present disclosure, and does not limit the present disclosure. Further, in each of the drawings, the same reference numerals are given to common configurations.

### [First Embodiment]

### <Microbial Test Kit>

As will be described later, the microbial test kit according to the first embodiment includes a reaction bottle 1 (reaction container), a waste liquid bottle 8 (waste liquid container), a luminescence measurement container 12, a sampling syringe 22 (first syringe), and a reagent-filled syringe 15 (second to fifth syringes).

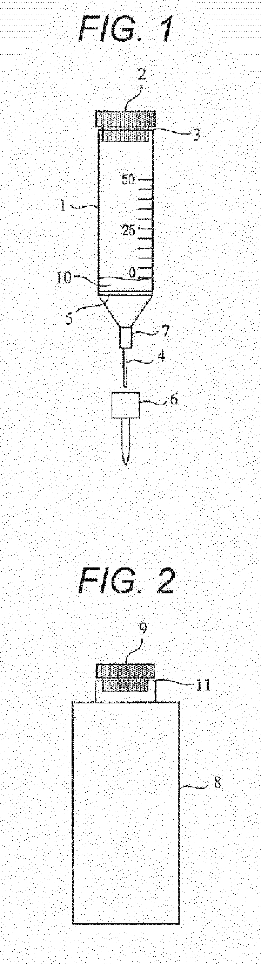

FIG. 1 is a schematic diagram illustrating the reaction bottle 1 of the microbial test kit according to a first embodiment. As illustrated in FIG. 1, the reaction bottle 1 (reaction container) has a sealing cap 2 (first sealing member), a nozzle 4, a filter 5, a nozzle cap 6, and a fitting portion 7.

The reaction bottle 1 has an opening 3 (first opening) at one end, and the sealing cap 2 is fitted to the opening 3. As the sealing cap 2, for example, a septum can be used. Examples of the material of the sealing cap 2 include natural rubber, butyl rubber, polytetrafluoroethylene (PTFE)/natural rubber, PTFE/butyl rubber, silicon/silicon rubber, PTFE/silicon, PTFE/silicon/PTFE, polyethylene, and Viton. With such a material, a nozzle (syringe needle) of various syringes described later can penetrate the sealing cap 2.

In the reaction bottle 1, an end portion opposite to the opening 3 is tapered toward the tip, and the nozzle 4 is connected to the tip. A fitting portion 7 is provided at a boundary between the reaction bottle 1 and the nozzle 4. The nozzle cap 6 covers the nozzle 4 so as to seal the reaction bottle 1 by fitting with the fitting portion 7. The nozzle cap 6 is detachable. The reaction bottle 1 is provided to a user in a state where the nozzle cap 6 is attached.

The materials of the reaction bottle 1 and the nozzle cap 6 can be a resin material such as polypropylene, polyethylene, or polystyrene, for example. The reaction bottle 1 can be manufactured by injection molding. The material of the nozzle 4 can be metal such as stainless steel (SUS 304), but may be resin. The outer diameter of the nozzle 4 can be, for example, 0.3 to 0.9 mmΦ, and the inner diameter can be, for example, 0.07 to 0.6 mm. The length of the nozzle 4 can be, for example, 13 to 38 mm. The tip of the nozzle 4 may be cut obliquely.

The filter 5 is disposed inside the reaction bottle 1 so as to partition the space between the opening 3 and the nozzle 4 into two spaces. The position of the filter 5 is such that a sufficient amount of specimen can be introduced between the opening 3 and the filter 5.

The filter 5 may be any filter as long as it can trap microorganisms to be inspected, and for example, a membrane filter can be used. Examples of the material of the filter 5 include cellulose mixed ester, PTFE, polyvinylidene fluoride (PVDF), polycarbonate, and the like.

The pore size of the filter 5 can be appropriately selected according to the size of the microorganism to be inspected and various conditions of the microbial test, and can be, for example, 0.20 µm or more. Specifically, examples of the filter 5 include membrane filters having pore sizes of 0.22 µm, 0.45 µm, and the like.

A reagent 10 is introduced in advance between the opening 3 and the filter 5. FIG. 1 illustrates a case where the reagent 10 is in a liquid form, but may be a powder. As the reagent 10, a medium component or an ATP eliminating reagent such as an ATP degrading enzyme may be stored alone, or a mixture thereof may be stored. In addition, the reagent 10 may contain components other than a medium component and an ATP eliminating reagent.

As the culture medium, for example, a soybean-casein digest culture medium, a thioglycolic acid culture medium, an R2A culture medium, a standard culture medium, a Sabouraud glucose culture medium, a Mosel enterobacteria enrichment broth medium, a Macconkey culture medium, a Rapaport bacilliase salmonella enrichment medium, a Celenite cystine culture medium, a tetrathionate culture medium, a Rapaport culture medium, a lactose broth medium, a Mueller-Hinton culture medium, or the like can be used according to the microorganism to be examined and various conditions of the microbial test.

At the time of performing the microbial test, the specimen is introduced into the space between the opening 3 and the filter 5. As illustrated in FIG. 1, a scale indicating the volume of the introduced specimen may be printed or engraved in the reaction bottle 1, whereby the liquid amount of the specimen can be easily grasped. When the reagent 10 is a liquid, a scale of 0 is set to the liquid level. When the reagent 10 is a powder, the scale is adjusted so that the liquid level is 0 when a certain amount of solvent is added.

As the volume of the reaction bottle 1, for example, a plurality of volumes such as a 1 mL specification, a 10 mL specification, a 50 mL specification, and a 100 mL specification may be included in the microbial test kit. As a result, the user can select the volume of the reaction bottle 1 according to the specimen used for the microbial test. The reaction bottle 1 illustrated in FIG. 1 has a 50 mL specification.

The inside of the reaction bottle 1 may be depressurized. In this case, by fitting the sealing cap 2 and the nozzle cap 6 under a reduced pressure environment, the inside of the reaction bottle 1 is sealed in a depressurized state. By removing the nozzle cap 6, the inside of the reaction bottle 1 becomes atmospheric pressure.

FIG. 2 is a schematic diagram illustrating the waste liquid bottle 8 (waste liquid container) of the microbial test kit according to the present embodiment. As illustrated in FIG. 2, a sealing cap 9 (second sealing member) is fitted into an opening 11 (second opening), and the inside of the waste liquid bottle 8 is decompressed and sealed. At the time of manufacturing the waste liquid bottle 8, the sealing cap 9 is fitted to the opening 11 under a reduced pressure environment. For example, a septum can be used as the sealing cap 9, and the material thereof is as described above.

The nozzle 4 of the reaction bottle 1 can penetrate the sealing cap 9, so that the liquid in the reaction bottle 1 can be filtered by the filter 5 by the pressure gradient (pressure difference) between the pressure in the reaction bottle 1 and the pressure in the waste liquid bottle 8, and collected in the waste liquid bottle 8. Therefore, the pressure inside the waste liquid bottle 8 may be reduced to such an extent that the liquid in the reaction bottle 1 can be discharged. When the liquid in the reaction bottle 1 is discharged, the nozzle cap 6 is removed to open the reaction bottle 1 to the atmosphere, so that the initial pressure in the waste liquid bottle 8 is set to be lower than the atmospheric pressure. Here, for example, assuming that the initial pressure inside the waste liquid bottle 8 is 0 atm and the liquid in the reaction bottle 1 is discharged to the waste liquid bottle 8 while a constant pressure is maintained, filtration of the liquid in the reaction bottle 1 by the filter 5 is performed under a constant pressure of 1 atm (atmospheric pressure).

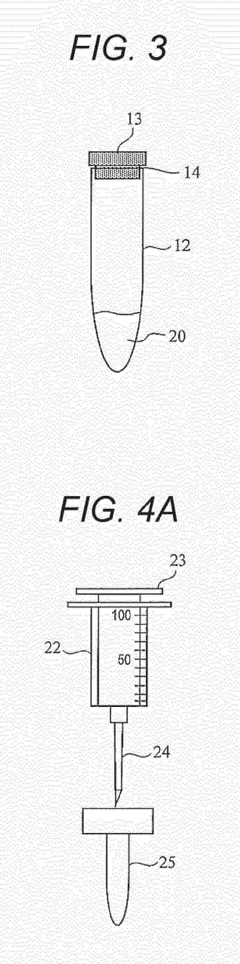

FIG. 3 is a schematic diagram illustrating the luminescence measurement container 12 of the microbial test kit according to the present embodiment. As illustrated in FIG. 3, a sealing cap 13 (third sealing member) is fitted to an opening 14 (third opening), and the inside of the luminescence measurement container 12 is decompressed and sealed. At the time of manufacturing the luminescence measurement container 12, the sealing cap 13 is fitted to the opening 14 under a reduced pressure environment. The pressure inside the luminescence measurement container 12 can be set in the same manner as the pressure inside the waste liquid bottle 8 described above. For example, a septum can be used as the sealing cap 13, and the material thereof is as described above.

A luminescent reagent 20 is previously introduced into the luminescence measurement container 12. The luminescent reagent 20 is a reagent that emits light by being mixed with ATP, and for example, a reagent containing luciferase and luciferin can be used. The luciferase may be introduced into the luminescent reagent 20, and the luciferin may be mixed with an ATP extraction reagent of the reagent-filled syringe 15 described later.

FIG. 4A is a schematic diagram illustrating the sampling syringe 22 (first syringe) of the microbial test kit according to the present embodiment. As illustrated in FIG. 4A, the sampling syringe 22 is a syringe capable of collecting a specimen, and includes a piston 23, a nozzle 24 (first syringe needle), and a nozzle cap 25. In the example illustrated in FIG. 4A, the sampling syringe 22 can collect a specimen up to 100 mL.

The nozzle 24 is provided to the user in a state of being covered with the nozzle cap 25. The nozzle cap 25 is detachable. The sampling syringe 22 is provided to the user in a state where the nozzle cap 25 is attached to the nozzle 24.

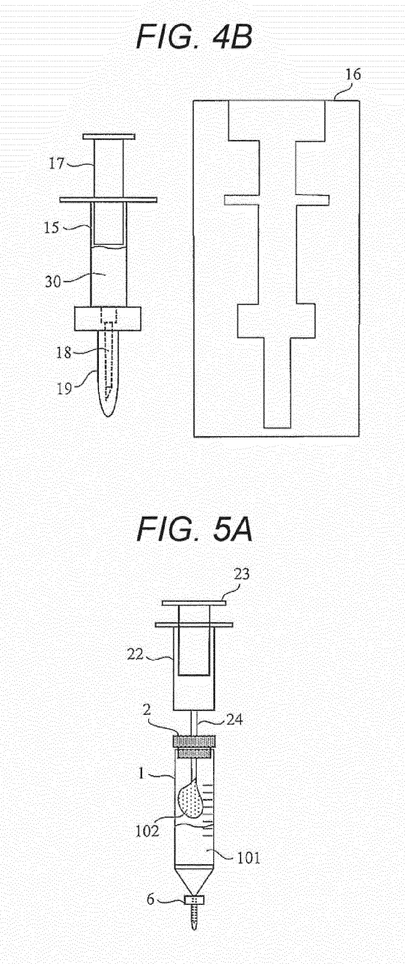

FIG. 4B is a schematic diagram illustrating the reagent-filled syringe 15 (second to fifth syringes) of the microbial test kit according to the present embodiment. As illustrated in FIG. 4B, the reagent-filled syringe 15 includes a piston 17, a nozzle 18 (second to fifth syringe needles), and a nozzle cap 19.

The reagent-filled syringe 15 is filled with an ATP test reagent 30 by a liquid amount required for one test. Examples of the ATP test reagent 30 include an ATP extraction reagent, an ATP eliminating reagent, a solvent, and a washing reagent. The reagent-filled syringe 15 is provided to the user with the ATP test reagent 30 introduced thereinto in advance for each type. The microbial test kit of the present embodiment includes the reagent-filled syringe 15 (second syringe) storing at least the ATP extraction reagent.

As the ATP extraction reagent, for example, an aqueous solution containing a surfactant such as benzalkonium chloride or benzethonium chloride, trichloroacetic acid (TCA), a Tris buffer, ethanol, a lytic enzyme having protease activity, lysozyme, or the like can be used.

When the reagent 10 in the reaction bottle 1 does not contain the ATP eliminating reagent, the reagent-filled syringe 15 (third syringe) containing the ATP eliminating reagent may be included in the microbial test kit.

When the reagent 10 is in a powder form, the reagent-filled syringe 15 (fourth syringe) storing the solvent of the reagent 10 may be included in the microbial test kit. As the solvent of the reagent 10, for example, a buffer solution usually used for a culture medium can be used.

Further, the reagent-filled syringe 15 (fifth syringe) containing the washing reagent may or may not be included in the microbial test kit. The washing reagent is used to wash the reaction bottle 1 and remove free ATP and ATP derived from dead bacteria (ATP other than ATP derived from living bacteria) in the specimen. As the washing reagent, for example, a buffer solution whose pH is adjusted to about 7 to 9 (for example, peptone solution, phosphate buffered saline, etc.), a buffer solution containing a surfactant that does not decompose microorganisms (for example, sodium lauryl sulfate), a buffer solution containing an enzyme such as a protease, or the like can be used.

The nozzle 18 is provided to the user in a state of being covered with the nozzle cap 19. The nozzle cap 19 is detachable. The reagent-filled syringe 15 is provided to the user in a state where the nozzle cap 19 is attached to the nozzle 18.

The reagent-filled syringe 15 may be provided to the user in a state of being housed in a syringe case 16, thereby preventing the ATP test reagent 30 from leaking due to unexpected pressing of the piston 17 before use.

The reaction bottle 1, the nozzle cap 6, the waste liquid bottle 8, the luminescence measurement container 12, the reagent-filled syringe 15, the sampling syringe 22, the pistons 17 and 23, and the nozzle caps 19 and 25 can be made of a resin material such as polypropylene, polyethylene, or polystyrene, for example, and can be manufactured by injection molding. Since the materials and sizes of the nozzles 18 and 24 can be similar to those of the nozzle 4 of the reaction bottle 1, the description thereof will be omitted.

The reaction bottle 1, the waste liquid bottle 8, the luminescence measurement container 12, the reagent-filled syringe 15, and the sampling syringe 22 may be provided to the user in a state where they are all sterilized. This makes it possible to prevent false positives and improve the detection accuracy of bacteria in a specimen. Examples of the sterilization method include high-pressure steam sterilization, boiling sterilization, distribution steam sterilization, ethylene oxide gas sterilization (EOG sterilization), radiation sterilization (gamma ray sterilization), and ultraviolet sterilization. In order to prevent deterioration of the material, EOG sterilization or gamma ray sterilization can be employed.

The reaction bottle 1, the luminescence measurement container 12, and the reagent-filled syringe 15 may be sterilized again after being filled with the reagent 10, the luminescent reagent 20, and the ATP test reagent 30, respectively. In general, since the luminescent reagent 20 and the ATP eliminating reagent contain an enzyme, EOG sterilization can be adopted as a sterilization method of the luminescence measurement container 12 and the reagent-filled syringe 15. Further, the reagent 10 contained in the reaction bottle 1 and the ATP test reagent 30 contained in the reagent-filled syringe 15 are also sterilized by filtration, so that sterility can be further secured.

Note that the microbial test kit of the present embodiment may include one reaction bottle 1, one waste liquid bottle 8, one luminescence measurement container 12, one reagent-filled syringe 15, and one sampling syringe 22, or may include a plurality of each. When a plurality of the components are included, components having different volumes or components having the same volume may be included.

### <Microbial Test Method>

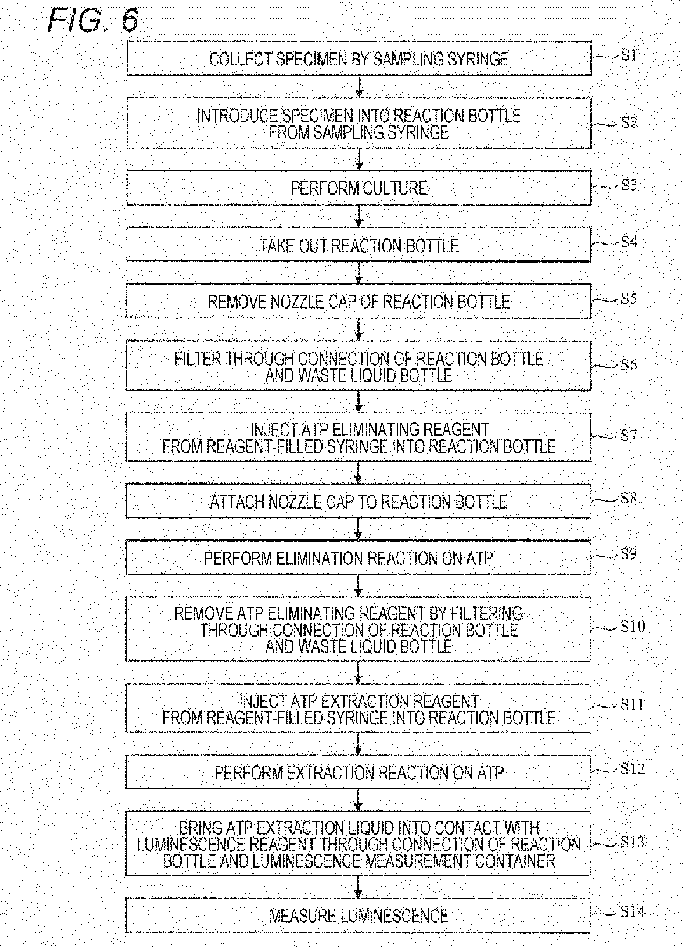

FIGS. 5A to 5I are diagrams illustrating a microbial test method using the microbial test kit according to the present embodiment, and FIG. 6 is a flowchart illustrating the microbial test method according to the present embodiment. In the present embodiment, a method in which a user manually performs a microbial test will be described.

The microbial management standard of pharmaceutical water is managed as a treatment standard value with an allowable amount of microorganisms contained in water based on the pharmacopoeia of each country. In the Japanese Pharmacopoeia, maintenance of a pharmaceutical water production line is required when normal water exceeds 100 CFU/mL, purified water exceeds 10 CFU/mL, and water for injection exceeds 10 CFU/100 mL. The treatment standard values in the United States Pharmacopeia are 500 CFU/mL for drinking water, 100 CFU/mL for purified water, and 10 CFU/100 mL for water for injection. The treatment standard values in the European Pharmacopeia are defined as 100 CFU/mL for purified water, 10 CFU/100 mL for highly purified water, and 10 CFU/100 mL for water for injection.

Therefore, in the following description, in order to perform a microbial test of pharmaceutical water in accordance with the test of the Japanese Pharmacopoeia, three specimens of normal water, purified water, and water for injection are used as the specimen 102, and one microbial test kit is used for each specimen 102. One microbial test kit includes the reaction bottle 1 pre-filled with 10 mL of the R2A liquid culture medium 101, the sampling syringe 22 (first syringe), a 200 mL waste liquid bottle 8a, a 10 mL waste liquid bottle 8b, a reagent-filled syringe 15a (third syringe) containing the ATP eliminating reagent 301, and a reagent-filled syringe 15b (second syringe) containing an ATP extraction reagent 302 as described later. In the microbial test kits for normal water and purified water, the capacity of the reaction bottle 1 is 1 mL, and in the microbial test kit for water for injection, the capacity of the reaction bottle 1 is 100 mL.

First, the user collects a certain amount of the specimen 102 using the sampling syringe 22 (Step S1). Here, in the case of normal water and in the case of purified water, 1 mL is collected, and in the case of water for injection, 100 mL is collected.

Next, as illustrated in FIG. 5A, the user causes the nozzle 24 (first syringe needle) of the sampling syringe 22 to penetrate the sealing cap 2 of the reaction bottle 1, and introduces the specimen 102 into the reaction bottle 1 (Step S2). At this time, when the inside of the reaction bottle 1 is not depressurized and is at atmospheric pressure, the user pushes the piston 23 to introduce the specimen 102 into the reaction bottle 1. When the reaction bottle 1 is depressurized, the specimen 102 is sucked by the pressure difference between the pressure inside the sampling syringe 22 and the pressure inside the reaction bottle 1 and introduced into the reaction bottle 1.

Since the sealing cap 2 is a septum formed of the above material, even if the nozzle 24 is penetrated and then the nozzle 24 is removed, the through hole is closed by the elastic force, so that the reaction bottle 1 can be kept sealed.

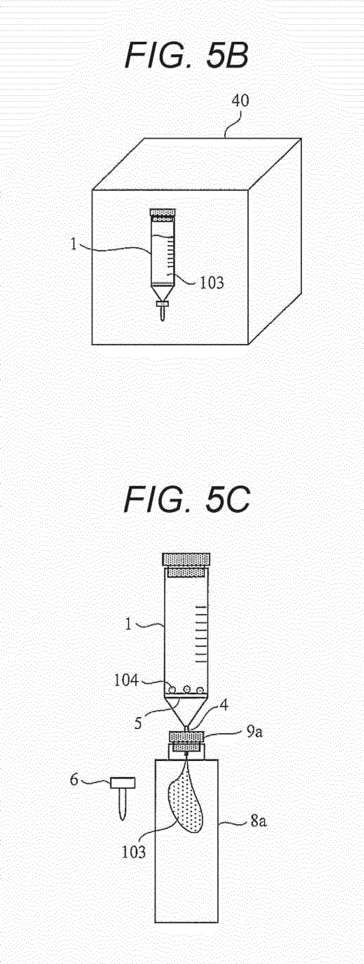

Next, as illustrated in FIG. 5B, the user removes the nozzle 24 of the sampling syringe 22 from the sealing cap 2, introduces the reaction bottle 1 containing a mixed liquid 103 of the specimen 102 and the R2A liquid culture medium 101 into an incubator 40 (thermostatic chamber), and performs culture for a certain period of time (Step S3). The temperature of the incubator 40 can be, for example, 25 to 37°C.

After culturing in the incubator 40 for the certain period of time, the user takes out the reaction bottle 1 from the incubator 40 (Step S4).

Next, as illustrated in FIG. 5C, the user removes the nozzle cap 6 from the reaction bottle 1 (Step S5), thereby opening the reaction bottle 1 to the atmosphere. Then, the user causes the nozzle 4 to penetrate the sealing cap 9a of the waste liquid bottle 8a. When the nozzle 4 communicates with the inside of the depressurized waste liquid bottle 8a in a sealed state, a pressure gradient is generated between the waste liquid bottle 8a and the reaction bottle 1 opened to the atmosphere. As a result, the mixed liquid 103 passes through the filter 5, is filtered, and moves to the waste liquid bottle 8a (Step S6). At this time, the filter 5 captures the microorganisms 104 (living bacteria) such as bacteria and fungi (mold or yeast) contained in the specimen 102. Free ATP and ATP derived from dead bacteria contained in the mixed liquid 103 basically flow to the waste liquid bottle 8a, but small amounts of the free ATP and the ATP derived from dead bacteria are captured by the filter 5.

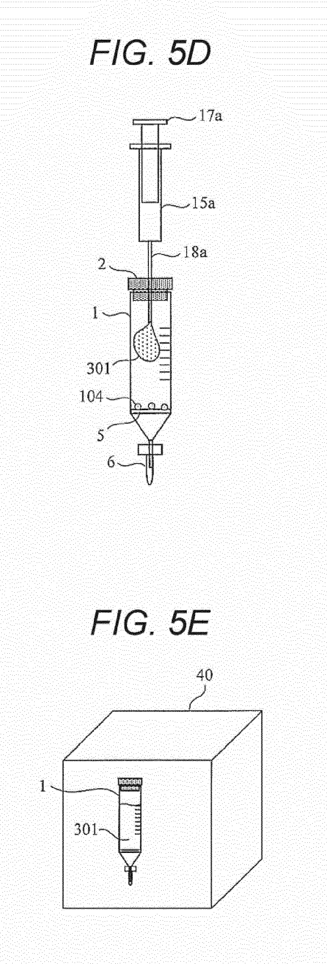

As illustrated in FIG. 5D, the user removes the nozzle 4 from the sealing cap 9a, causes the nozzle 18a (third syringe needle) of the reagent-filled syringe 15a filled with the ATP eliminating reagent 301 to penetrate the sealing cap 2 of the reaction bottle 1, and pushes the piston 17a to inject it into the reaction bottle 1 (Step S7) . Further, the user attaches the nozzle cap 6 to the reaction bottle 1 (Step S8).

Next, as illustrated in FIG. 5E, the user removes the nozzle 18a of the reagent-filled syringe 15a from the sealing cap 2, introduces the reaction bottle 1 into the incubator 40, and performs an elimination reaction in the range of room temperature to 37°C for about 30 minutes after the injection of the ATP eliminating reagent 301 (Step S9). At this time, the reaction may be performed in a state of being left or may be performed while being shaken. It is not limited to the reaction in the incubator 40: the reaction bottle 1 may be placed in a temperature-controlled space such as a heat block to cause a reaction, or may be left at room temperature.

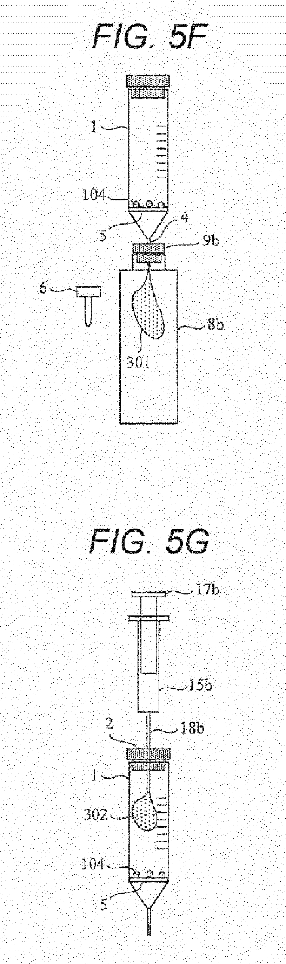

After the ATP eliminating reaction for 30 minutes, as illustrated in FIG. 5F, the user takes out the reaction bottle 1 from the incubator 40. Then, the user removes the nozzle cap 6 of the reaction bottle 1 and causes the nozzle 4 to penetrate the sealing cap 9b of the waste liquid bottle 8b to moves the ATP eliminating reagent 301 to the waste liquid bottle 8b (Step S10). At this time, since the inside of the waste liquid bottle 8b is depressurized, the ATP eliminating reagent 301 is sucked by the pressure difference between the inside of the reaction bottle 1 and the inside of the waste liquid bottle 8b.

As described above, by using the two different waste liquid bottles 8a and 8b for the filtration of the specimen 102 and the disposal of the ATP eliminating reagent 301, it is possible to prevent contamination of the nozzle 4 from bacteria attached to the sealing caps 9a and 9b and ATP floating in the environment. When one waste liquid bottle 8 is repeatedly used, it is possible to prevent contamination by changing the position where the nozzle 4 penetrates the sealing cap 9.

As illustrated in FIGS. 5D to 5F, by introducing the ATP eliminating reagent 301 into the reaction bottle 1 and decomposing and removing free ATP and ATP derived from dead bacteria trapped in the filter 5, the accuracy of the microbial test can be further improved.

Next, as illustrated in FIG. 5G, the user removes the nozzle 4 from the sealing cap 9b of the waste liquid bottle 8b, causes a nozzle 18b (second syringe needle) of the reagent-filled syringe 15b filled with the ATP extraction reagent 302 to penetrate the sealing cap 2 of the reaction bottle 1. Then, the user presses the piston 17b to inject the ATP extraction reagent 302 into the reaction bottle 1 (Step S11).

After the injection of the ATP extraction reagent 302, the reaction is performed at room temperature for about 1 minute, whereby viable bacteria captured by the filter 5 are disrupted, and ATP released by being lysed is dispersed in the ATP extraction reagent 302, whereby an ATP extraction liquid 105 is obtained (Step S12).

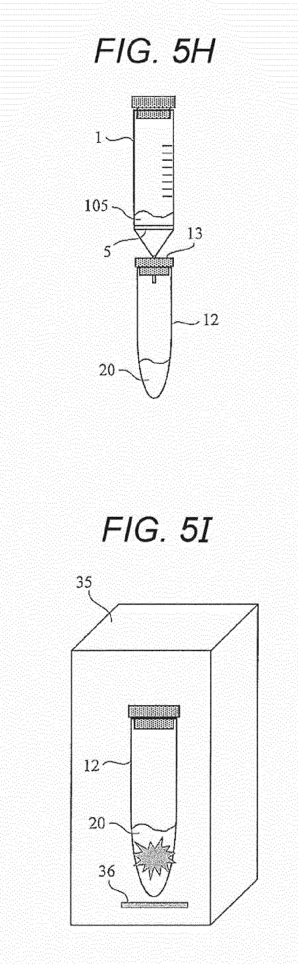

Next, as illustrated in FIG. 5H, the user removes the nozzle 18b of the reagent-filled syringe 15b from the sealing cap 2 and causes the nozzle 4 of the reaction bottle 1 to penetrate the sealing cap 13 of the luminescence measurement container 12 to moves the ATP extraction liquid 105 to the luminescence measurement container 12 (Step S13). At this time, since the inside of the luminescence measurement container 12 is depressurized, the ATP extraction liquid 105 is sucked, passes through the filter 5, and is introduced into the luminescence measurement container 12. When the ATP extraction liquid 105 comes into contact with the luminescent reagent 20 previously introduced into the luminescence measurement container 12, bioluminescence occurs.

As illustrated in FIG. 5I, the user installs the luminescence measurement container 12 into which the ATP extraction liquid 105 is introduced in a luminescence measurement device 35. A detector 36 of the luminescence measurement device 35 measures the amount of light generated by the luminescence reaction between the luminescent reagent 20 and ATP derived from viable bacteria (Step S14). As the luminescence measurement device 35, any device capable of detecting the amount of luminescence of the luminescent reagent 20 may be used, and for example, a luminometer or the like can be used. As the detector 36, for example, a photomultiplier tube is used. Luminescence measurement can be performed by a photon counting method for digitally processing a signal of the photomultiplier tube. The luminescence measurement device 35 may include a display (not illustrated) that displays a result of the luminescence measurement.

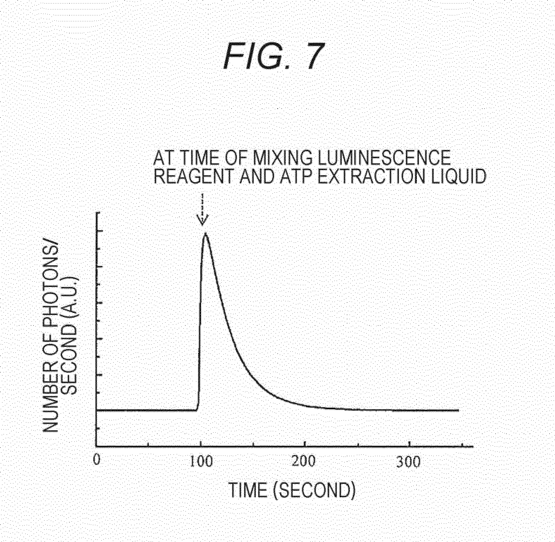

FIG. 7 is a graph illustrating an example of the photon counting time course obtained by the detector 36 of the luminescence measurement device 35. The amount of ATP can be calculated by integrating the amount of light emission for a certain period of time in the photon counting time course or by normalizing an average value of the amount of light emission as a relative light unit (RLU). The user can determine that a microorganism is present when the amount of ATP exceeds a certain threshold.

The microbial test using the microbial test kit of the present embodiment can also be performed in a clean bench or a safety cabinet. As a result, false positives can be more reliably prevented, and the accuracy of the test can be improved.

### <Technical Effect>

As described above, the microbial test kit of the present embodiment includes the sealed reaction bottle 1 having the filter 5 therein, the sampling syringe 22 capable of collecting a specimen, the waste liquid bottle 8 sealed under reduced pressure, the luminescence measurement container 12 sealed under reduced pressure and containing the luminescent reagent 20, and the reagent-filled syringe 15 containing the ATP test reagent 30. With such a configuration, the aseptic manipulation can be easily performed in the microbial test by the ATP method. Therefore, a highly accurate microbial test can be easily performed.

### [Second Embodiment]

In the first embodiment, the microbial test method has been described in which after a specimen is introduced into the reaction bottle 1, ATP derived from viable bacteria is extracted after culturing is performed, and luminescence measurement is performed. The second embodiment is different from the first embodiment in that after the specimen is introduced into the reaction bottle 1, ATP derived from viable bacteria is extracted without culturing.

### <Microbial Test Kit>

Since the microbial test kit according to the second embodiment is similar to that of the first embodiment, the description thereof is omitted.

### <Microbial Test Method>

In a microbial test method of the second embodiment, unlike the first embodiment, Steps S3 and S4 (FIG. 5B) illustrated in FIG. 6 are not performed. Thus, by omitting the culture of the specimen, it is possible to quickly detect ATP derived from a trace amount of viable bacteria in the specimen.

In the present embodiment, since the specimen is not cultured, the reagent 10 may not be contained in the reaction bottle 1. Accordingly, the cost of the reagent 10 can be reduced.

In the present embodiment, since the specimen is not cultured, it is necessary to detect an extremely small amount of ATP derived from viable bacteria. Therefore, the microbial test method of the present embodiment may have a step of washing the reaction bottle 1 using the reagent-filled syringe 15 (fifth syringe) filled with a washing reagent as the ATP test reagent 30 in order to remove free ATP and ATP derived from dead bacteria (ATP other than ATP derived from living bacteria) in the specimen from the reaction bottle 1.

The washing of the reaction bottle 1 with the washing reagent can be performed, for example, between Steps S6 and S7 (before the ATP elimination reaction) or between Steps S10 and S11 (after the ATP elimination reaction) illustrated in FIG. 6 by the same method as in the case of the ATP eliminating reagent. As described above, the reliability of the microbial test can be further improved by washing ATP other than ATP derived from viable bacteria.

### <Technical Effect>

As described above, in the present embodiment, in the microbial test method using the microbial test kit according to the first embodiment, the culture of the microorganism in the specimen is omitted, so that the highly reliable microbial test can be performed more quickly.

### [Third Embodiment]

### <Microbial Test Kit>

In the first embodiment, the case where the reagent 10 contained in the reaction bottle 1 is liquid has been described. A microbial test kit according to a third embodiment is different from that of the first embodiment in that the reagent 10 contained in the reaction bottle 1 is a powdery culture medium, and the reagent-filled syringe 15 (fourth syringe) in which a solvent of the reagent 10 is contained is further provided as the ATP test reagent 30. As the solvent of the reagent 10, for example, a buffer solution usually used for a culture medium can be used.

<Microbial Test Method>

The microbial test method of the third embodiment is different from the microbial test method according to the first embodiment in further including a step of dissolving a powdery reagent 10 in a solvent. Specifically, in the present embodiment, before Step S1 illustrated in FIG. 6, the user introduces the solvent into the reaction bottle 1 from a reagent-filled syringe (not illustrated) containing the solvent, and dissolves the solvent by leaving or stirring the solvent at room temperature for about 5 to 30 minutes until the solvent is homogenized, thereby obtaining a liquid culture medium. Thereafter, each step illustrated in FIG. 6 is performed.

Also in the present embodiment, the culture of the specimen (Steps S3 and S4) may be omitted as in the second embodiment.

### <Technical Effect>

As described above, in the present embodiment, the reagent 10 contained in the reaction bottle 1 is the powdery culture medium. This makes it possible to preserve the medium for a longer period of time than in the case of a liquid medium.

### [Fourth Embodiment]

In the first to third embodiments, the method in which the user manually performs the microbial test using the microbial test kit has been described. In the present embodiment, the microbial test is automatically performed by the microbial test device including drivers of each configuration of the microbial test kit.

### <Microbial Test Device>

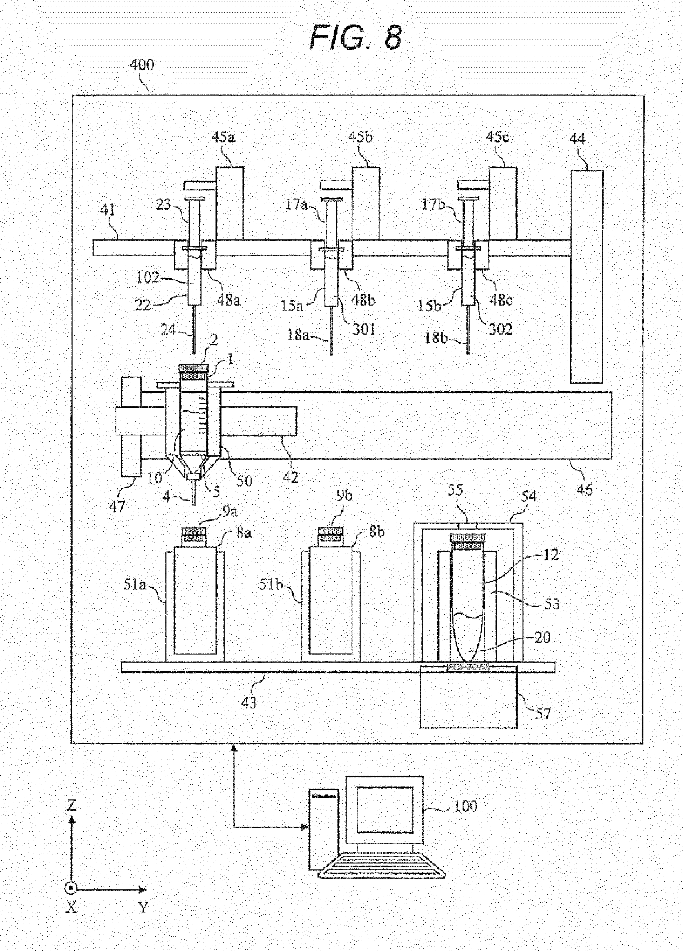

FIG. 8 is a schematic diagram illustrating a configuration of a microbial test device 400 according to a fourth embodiment. The microbial test device 400 of the present embodiment is an automated device including the microbial test kit of the first embodiment.

As illustrated in FIG. 8, the microbial test device 400 includes an installation table 41 (first installation table) arranged in an upper stage, an installation table 42 (second installation table) arranged in a middle stage, and an installation table 43 (third installation table) arranged in a lower stage.

The installation table 41 is arbitrarily movable in the Z direction by an actuator 44 (first driver). Syringe holders 48a to 48c are arranged on the installation table 41 along the Y direction. The syringe holder 48a holds the sampling syringe 22 (first syringe), the syringe holder 48b holds the reagent-filled syringe 15a (third syringe), and the syringe holder 48c holds the reagent-filled syringe 15b (second syringe).

Further, on the installation table 41, an actuator 45a (second driver) that drives the piston 23 of the sampling syringe 22, an actuator 45b (second driver) that drives the piston 17a of the reagent-filled syringe 15a, and an actuator 45c (second driver) that drives the piston 17b of the reagent-filled syringe 15b are installed along the Y direction.

The installation table 42 is arbitrarily movable in the Y direction and the Z direction by actuators 46 and 47 (first driver). The installation table 42 is provided with a reaction bottle holder 50 that holds the reaction bottle 1. The reaction bottle holder 50 may include a heater (not illustrated) that adjusts the temperature inside the reaction bottle 1. Although not illustrated, the container containing the specimen 102 is placed at a position where the specimen 102 can be collected by the sampling syringe 22 on the installation table 42.

The installation table 43 is provided with a waste liquid bottle holder 51a holding the waste liquid bottle 8a, a waste liquid bottle holder 51b holding the waste liquid bottle 8b, a luminescence measurement container holder 53 holding the luminescence measurement container 12, a light shielding case 54, and a luminescence measurement device 57. The luminescence measurement device 57 is installed at the bottom of the luminescence measurement container holder 53 so as to be able to detect luminescence from the luminescence measurement container 12. When luminescence measurement is performed, the light shielding case 54 is installed so as to cover the luminescence measurement container holder 53 and the luminescence measurement container 12 after the luminescence measurement container is set in the luminescence measurement container holder. The ceiling portion of the light shielding case 54 is provided with a slit 55 through which the nozzle 4 of the reaction bottle 1 can pass.

The microbial test device 400 may have a cap attachment/detachment mechanism (not illustrated) for attaching/detaching the nozzle cap 6 of the reaction bottle 1, the nozzle caps of the reagent-filled syringes 15a and 15b, and the nozzle cap 25 of the sampling syringe 22.

The microbial test device 400 is connected to the control device 100 (controller) in a wireless or wired manner. The control device 100 is, for example, a computer terminal such as a personal computer, a tablet, or a smartphone, and is programmed to control driving of each component of the microbial test device 400 to automatically perform each step in FIG. 6.

Although not illustrated, the control device 100 includes an arithmetic unit, a storage, a display, and the like. The arithmetic unit obtains the amount of luminescence from the detection signal received from the luminescence measurement device 57, and calculates the amount of ATP in the specimen 102 based on the amount of luminescence. In addition, the arithmetic unit compares the calculated amount of luminescence or the amount of ATP with a certain threshold to determine whether the specimen is positive (bacteria are present in the specimen) or negative (bacteria are not present in the specimen). The storage stores various data such as the certain threshold of the amount of luminescence or the amount of ATP. The display displays the calculated amount of luminescence and amount of ATP, a determination result as to whether the specimen is positive or negative, and the like.

The microbial test device 400 may be installed in a clean bench or a safety cabinet. As a result, false positives can be more reliably prevented, and the accuracy of the test can be improved.

### <Microbial Test Method>

The microbial test method of the present embodiment is similar to the method illustrated in FIG. 6, but is different from that of the first embodiment in that the operation of each component of the microbial test device 400 is automatically controlled by the control device 100 to perform the microbial test. Hereinafter, the microbial test method of the present embodiment will be described along each step illustrated in FIG. 6.

In Step S1, the control device 100 drives the actuator 46 to move a specimen container (not illustrated) placed on the installation table 42 below the sampling syringe 22. Next, the control device 100 drives the actuator 44 downward to immerse the nozzle 24 in the specimen 102, and drives the actuator 45a to move the piston 23, thereby collecting a certain amount of the specimen 102 in the sampling syringe 22. Thereafter, the control device 100 drives the actuator 44 upward to retract the sampling syringe 22 from the specimen container.

In Step S2, the control device 100 drives the actuator 46 to move the reaction bottle 1 below the sampling syringe 22. Next, the control device 100 drives the actuator 44 downward to cause the nozzle 24 to penetrate the sealing cap 2 of the reaction bottle 1. At this time, when the inside of the reaction bottle 1 is not depressurized and is at atmospheric pressure, the control device 100 drives the actuator 45a to push the piston 23, thereby introduces the specimen 102 into the reaction bottle 1. When the reaction bottle 1 is depressurized, the specimen 102 is sucked by the pressure difference between the sampling syringe 22 and the reaction bottle 1 and introduced into the reaction bottle 1.

Next, in Step S3, the control device 100 drives the actuator 44 upward to remove the nozzle 24 from the sealing cap 2. Thereafter, the control device 100 adjusts the temperature of the reaction bottle holder 50 to, for example, 25 to 37°C and performs culture for a certain period of time.

After culturing for the certain period of time, the control device 100 stops temperature control by the reaction bottle holder 50 instead of Step S4.

In Step S5, the control device 100 drives a cap attachment/detachment mechanism (not illustrated) to remove the nozzle cap 6 from the reaction bottle 1.

In Step S6, the control device 100 drives the actuator 47 downward to cause the nozzle 4 to penetrate the sealing cap 9a of the waste liquid bottle 8a. When the nozzle 4 communicates with the inside of the depressurized waste liquid bottle 8a in a sealed state, a pressure gradient is generated between the nozzle 4 and the reaction bottle 1 opened to the atmosphere. As a result, the mixed liquid of the specimen 102 and the reagent 10 passes through the filter 5, is filtered, and moves to the waste liquid bottle 8a. At this time, the filter 5 captures microorganisms such as bacteria and fungi (mold or yeast) contained in the specimen 102.

In Step S7, the control device 100 drives the actuator 47 upward to remove the nozzle 4 from the sealing cap 9a of the waste liquid bottle 8a, and drives the actuator 46 in the Y direction to move the reaction bottle 1 below the reagent-filled syringe 15a. Next, the control device 100 drives the actuator 44 downward to cause the nozzle 18a of the reagent-filled syringe 15a to penetrate the sealing cap 2. Next, the control device 100 drives the actuator 45b to press the piston 17a of the reagent-filled syringe 15a, thereby a certain amount of the ATP eliminating reagent 301 is injected into the reaction bottle 1.

In Step S8, the control device 100 drives the cap attachment/detachment mechanism to attach the nozzle cap 6 to the reaction bottle 1.

In Step S9, the control device 100 drives the actuator 44 upward to remove the nozzle 18a of the reagent-filled syringe 15a from the sealing cap 2. Next, the control device 100 adjusts the temperature of the reaction bottle holder 50, and for example, an elimination reaction is performed in the range of room temperature to 37°C for 30 minutes after the injection of the ATP eliminating reagent 301.

In Step S10, after the ATP elimination reaction for 30 minutes, the control device 100 drives the cap attachment/detachment mechanism to remove the nozzle cap 6 of the reaction bottle 1, and drives the actuator 47 downward to cause the nozzle 4 to penetrate the sealing cap 9b of the waste liquid bottle 8b. As a result, the ATP eliminating reagent 301 is moved to the waste liquid bottle 8b.

In Step S11, the control device 100 drives the actuator 47 upward to remove the nozzle 4 from the sealing cap 9b of the waste liquid bottle 8b. Next, the control device 100 drives the actuator 46 to move the reaction bottle 1 below the reagent-filled syringe 15b, drives the actuator 47 downward to cause the nozzle 18b of the reagent-filled syringe 15b to penetrate the sealing cap 2, and drives the actuator 45c downward to press the piston 17b. Thus, a certain amount of the ATP extraction reagent 302 is injected into the reaction bottle 1.

In Step S12, after the injection of the ATP extraction reagent 302, the reaction is performed at room temperature for about 1 minute, whereby viable bacteria captured by the filter 5 are disrupted, and ATP released by being lysed is dispersed in the ATP extraction reagent 302, whereby an ATP extraction liquid 105 is obtained.

In Step S13, the control device 100 drives the actuator 47 downward to cause the nozzle 4 to penetrate the sealing cap 13 of the luminescence measurement container 12. At this time, the light shielding case 54 covers the luminescence measurement container 12 and the luminescence measurement container holder 53, and the nozzle 4 passes through the slit 55. Accordingly, the ATP extraction liquid 105 moves to the luminescence measurement container 12. At this time, the control device 100 causes the luminescence measurement device 57 to start luminescence measurement. When the ATP extraction liquid 105 comes into contact with the luminescent reagent 20 previously introduced into the luminescence measurement container 12, bioluminescence occurs.

In Step S14, the luminescence measurement device 57 detects luminescence of the luminescent reagent 20 and outputs a detection signal to the control device 100. The control device 100 calculates the amount of luminescence based on the received detection signal and calculates the amount of ATP in the specimen 102. In addition, the control device 100 compares the calculated amount of luminescence or the amount of ATP with a certain threshold stored in advance in the storage, and determines whether the specimen is positive (bacteria are present in the specimen) or negative (bacteria are not present in the specimen). The determination result may be output to a display or the like.

Note that, in the present embodiment, the installation table 41 is moved by the actuator 44, and the installation table 42 is moved by the actuators 46 and 47, but the present invention is not limited thereto as long as the relative positions of the reaction bottle 1, the sampling syringe 22, the reagent-filled syringes 15a and 15b, the waste liquid bottles 9a and 9b, and the luminescence measurement container 9 can be changed. For example, the installation table 42 on which the reaction bottle 1 is installed may be fixed, and the installation tables 41 and 43 may move. Alternatively, the installation tables 41 and 43 may be fixed so that only the installation table 42 moves in the vertical direction and the horizontal direction.

### <Technical Effect>

As described above, the microbial test device 400 of the present embodiment has a configuration in which the microbial test using the microbial test kit according to the first embodiment is automated. This makes it possible to more easily and more quickly perform a highly reliable microbial test.

### [Fifth Embodiment]

### <Microbial Test Device>

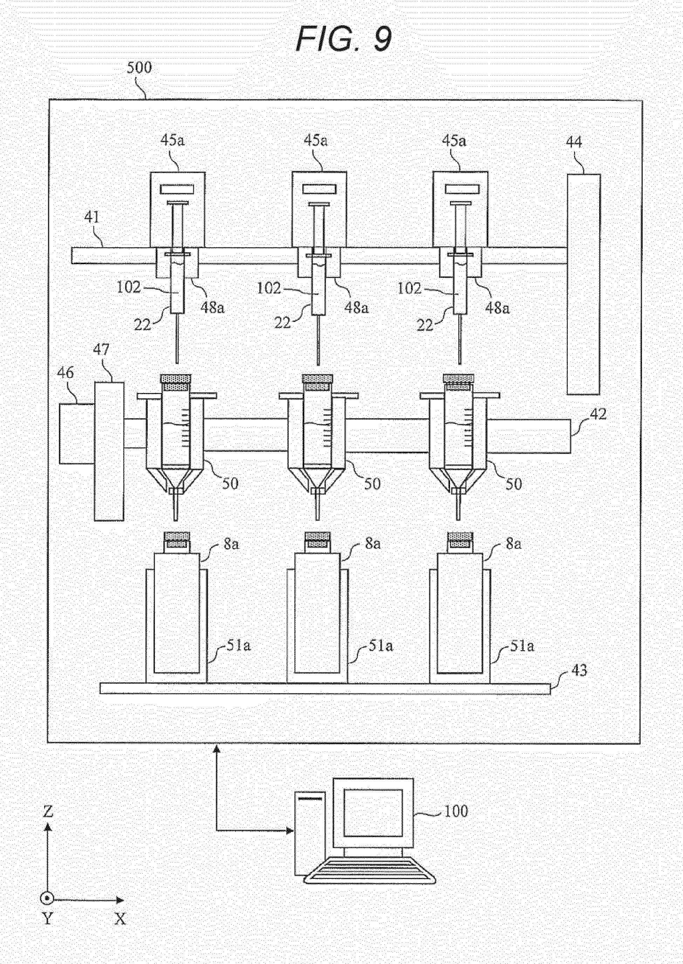

FIG. 9 is a schematic diagram illustrating a configuration of a microbial test device 500 according to a fifth embodiment. The microbial test device 500 of the present embodiment is different from that of the fourth embodiment in that a plurality of microbial test kits are arranged in the X direction.

In the microbial test device 500 illustrated in FIG. 9, three of each component of the microbial test kit are arranged in the X direction. Three of the actuators 45a to 45c, the syringe holders 48a to 48c, the reaction bottle holder 50, the waste liquid bottle holders 51a and 51b, and the luminescence measurement container holder 53 are also arranged in the X direction, but only the actuator 45a, the syringe holder 48a, the reaction bottle holder 50, and the waste liquid bottle holder 51a located in front are illustrated.

### <Technical Effect>

As described above, the microbial test device 500 of the present embodiment is a device that automates the microbial test using the microbial test kit according to the first embodiment, and can simultaneously perform the microbial test on the plurality of specimens 102. As a result, a reliable microbial test can be more easily and more quickly performed, and the throughput of the microbial test can be improved.

### [Modifications]

The present disclosure is not limited to the examples described above, but includes various modifications. For example, the above embodiments have been described in detail for easy understanding of the present disclosure, and the invention does not necessarily have all the configurations described. In addition, some of certain embodiment can be replaced with the configuration of the other embodiment. Further, it is possible to add the configuration of one embodiment to the configuration of another embodiment. It is also possible to add, delete, or replace a part of the configuration of another embodiment with respect to a part of the configuration of each embodiment.

In the fourth and fifth embodiments, the microbial test device that automatically performs the microbial test method according to the first embodiment has been described. As a modification, for example, the microbial test method according to the second or third embodiment may be automatically performed.

### Reference Signs List

- 1: reaction bottle
- 2: sealing cap
- 3: opening
- 4: nozzle
- 5: filter
- 6: nozzle cap
- 7: fitting portion
- 8, 8a, 8b: waste liquid bottle
- 9, 9a, 9b: sealing cap
- 10: reagent
- 11: opening
- 12: luminescence measurement container
- 13: sealing cap
- 14: opening
- 15, 15a, 15b: reagent-filled syringe
- 16: syringe case
- 17, 17a, 17b: piston
- 18, 18a, 18b: nozzle
- 19: nozzle cap
- 20: luminescent reagent
- 22: sampling syringe
- 23: piston
- 24: nozzle
- 25: nozzle cap
- 30: ATP test reagent
- 35: luminescence measurement device
- 36: detector
- 40: incubator
- 41 to 43: installation table
- 44, 45a to 45c, 46, 47: actuator
- 48a: to 48c syringe holder
- 50: reaction bottle holder
- 51a, 51b: waste liquid bottle holder
- 53: luminescence measurement container holder
- 54: light shielding case
- 55: slit
- 57: luminescence measurement device
- 100: control device
- 101: R2A liquid culture medium
- 102: specimen
- 103: mixed liquid
- 104: microorganism
- 105: ATP extraction liquid
- 301: ATP eliminating reagent
- 302: ATP extraction reagent
- 400, 500: microbial test device

## Claims

1. A microbial test kit comprising:
a first syringe capable of collecting a specimen and having a first syringe needle;
a second syringe containing an ATP extraction reagent and having a second syringe needle;
a sealed reaction container including a first opening, a first sealing member fitted to the first opening, a nozzle, a nozzle cap covering the nozzle, and a filter disposed so as to partition the first opening and the nozzle;
a waste liquid container including a second opening and a second sealing member fitted to the second opening, and sealed under reduced pressure; and
a luminescence measurement container that includes a third opening and a third sealing member fitted to the third opening, contains a luminescent reagent that emits light in presence of ATP, and is sealed under reduced pressure.

2. The microbial test kit according to claim 1, wherein the first syringe needle and the second syringe needle are capable of penetrating the first sealing member while keeping the reaction container sealed,
the nozzle is capable of penetrating the second sealing member while keeping the waste liquid container sealed, and
the nozzle is capable of penetrating the third sealing member while keeping the luminescence measurement container sealed.

3. The microbial test kit according to claim 1, further comprising a third syringe containing an ATP eliminating reagent and having a third syringe needle,
wherein the third syringe needle is capable of penetrating the first sealing member while keeping the reaction container sealed.

4. The microbial test kit according to claim 1, wherein an inside of the reaction container is depressurized.

5. The microbial test kit according to claim 1, wherein a pore size of the filter is 0.20 µm or more.

6. The microbial test kit according to claim 1, wherein a reagent containing a medium component is stored in the reaction container.

7. The microbial test kit according to claim 6, wherein the reagent further contains an ATP eliminating reagent.

8. The microbial test kit according to claim 6, wherein the reagent is in a liquid state.

9. The microbial test kit according to claim 6, wherein
the reagent is in a powder form,
the microbial test kit further comprising a fourth syringe containing a solvent of the reagent and having a fourth syringe needle.

10. The microbial test kit according to claim 1, further comprising a fifth syringe containing a washing reagent and having a fifth syringe needle.

11. A microbial test method comprising:
preparing the microbial test kit according to claim 1;
collecting the specimen with the first syringe;
causing the first syringe needle to penetrate the first sealing member to introduce the specimen into the reaction container;
causing the nozzle to penetrate the second sealing member to filter the specimen with the filter by a pressure difference between the reaction container and the waste liquid container to capture microorganisms in the specimen;
causing the second syringe needle to penetrate the first sealing member and introducing the ATP extraction reagent into the reaction container to extract ATP from the microorganisms; and
causing the nozzle to penetrate the third sealing member to introduce the extraction liquid of ATP into the luminescence measurement container by a pressure difference between the reaction container and the luminescence measurement container and bring the extraction liquid of ATP into contact with the luminescent reagent.

12. The microbial test method according to claim 11, wherein
the microbial test kit further includes:
a third syringe containing an ATP eliminating reagent and having a third syringe needle,
the microbial test method further comprising:
after filtering the specimen with the filter to capture microorganisms in the specimen, causing the third syringe needle to penetrate the first sealing member and introducing the ATP eliminating reagent into the reaction container; and
causing the nozzle to penetrate the second sealing member to discharge the ATP eliminating reagent to the waste liquid container by a pressure difference between the reaction container and the waste liquid container.

13. The microbial test method according to claim 11, wherein
the reaction container contains a reagent containing a medium component, and
the microbial test method further comprising:
culturing the specimen after introducing the specimen into the reaction container.

14. The microbial test method according to claim 13, wherein
the reagent is in a powder form, and the microbial test kit further includes a fourth syringe containing a solvent of the reagent and having a fourth syringe needle,
the microbial test method further comprising:
causing the fourth syringe needle to penetrate the first sealing member and introducing the solvent into the reaction container before introducing the specimen into the reaction container.

15. The microbial test method according to claim 11, wherein
the microbial test kit further includes a fifth syringe containing a washing reagent and having a fifth syringe needle,
the microbial test method further comprising:
filtering the specimen with the filter to capture microorganisms in the specimen, then causing the fifth syringe needle to penetrate the first sealing member, and introducing the washing reagent into the reaction container; and
causing the nozzle to penetrate the second sealing member to discharge the washing reagent to the waste liquid container by a pressure difference between the reaction container and the waste liquid container.

16. The microbial test method according to claim 12, wherein
the microbial test kit further includes a fifth syringe containing a washing reagent and having a fifth syringe needle,
the microbial test method further comprising:
after discharging the ATP eliminating reagent into the waste liquid container, causing the fifth syringe needle to penetrate the first sealing member and introducing the washing reagent into the reaction container; and
causing the nozzle to penetrate the second sealing member to discharge the washing reagent to the waste liquid container by a pressure difference between the reaction container and the waste liquid container.

17. A microbial test device comprising:
the microbial test kit according to claim 1;
a first driver configured to change relative positions between the reaction container, and the first syringe, the second syringe, the waste liquid container, and the luminescence measurement container;
a second driver configured to drive the first syringe and the second syringe;
a luminescence measurement device configured to detect luminescence from the luminescence measurement container; and
a controller configured to control driving of the first driver and the second driver.

18. The microbial test device according to claim 17, wherein
the controller is configured to:
drive the second driver to collect the specimen with the first syringe;
drive the first driver to cause the first syringe needle to penetrate the first sealing member, and drive the second driver to introduce the specimen into the reaction container;
drive the first driver to cause the nozzle to penetrate the second sealing member to filter the specimen with the filter by a pressure difference between the reaction container and the waste liquid container and capture microorganisms in the specimen;
drive the first driver to cause the second syringe needle to penetrate the first sealing member, drive the second driver to introduce the ATP extraction reagent into the reaction container and extract ATP from the microorganisms; and
drive the first driver to cause the nozzle to penetrate the third sealing member to introduce the extraction liquid of ATP into the luminescence measurement container by a pressure difference between the reaction container and the luminescence measurement container and bring the extraction liquid of ATP into contact with the luminescent reagent.

19. The microbial test device according to claim 17, wherein
the microbial test kit further includes a third syringe containing an ATP eliminating reagent and having a third syringe needle,
the first driver further changes a relative position between the reaction container and the third syringe,
the second driver further drives the third syringe, and
the controller is configured to:
after filtering the specimen with the filter to capture microorganisms in the specimen, drive the first driver to cause the third syringe needle to penetrate the first sealing member, and drive the second driver to introduce the ATP eliminating reagent into the reaction container; and
drive the first driver to cause the nozzle to penetrate the second sealing member to discharge the ATP eliminating reagent to the waste liquid container by a pressure difference between the reaction container and the waste liquid container.

20. The microbial test device according to claim 17, further comprising:
a first installation table on which the first syringe and the second syringe are installed;
a second installation table disposed below the first installation table and on which the reaction container is installed; and
a third installation table disposed below the second installation table and on which the waste liquid container and the luminescence measurement container are placed,
wherein the first driver drives one or more of the first installation table, the second installation table, and the third installation table.
